# EUROPEAN PATENT APPLICATION

(11) **EP 2 740 422 A1**
(43) Date of publication of application: **11.06.2014**
(21) Application number: 12195760.9
(22) Date of filing: 05.12.2012
(51) Int. Cl.: A61B 17/34

(54) **Safety neural injection system and related methods**

(71) Applicant: Custom Medical Applications, Irving, TX 75063 (US)
(72) Inventor: Racz, N. Sandor, Coppell, TX Texas 75019 (US)
(74) Representative: Selby, David Sean

(57) **Abstract**

Various embodiments of the present invention generally relate to safety neural injection systems and related methods of use comprising a cannula, with or without a side port, and a stylet, defined by a first inside diameter, a first outside diameter, a first length, and an open distal end in fluid communication with the inside and outside of the hollow cannula wherein the medicament is administered with the stylet inserted at least partially within the cannula.

## Description

### FIELD

The present invention generally relates to safety neural injection systems and related methods of use.

### BACKGROUND

Needles and needle systems are used extensively in a wide variety of procedures which are performed in various fields of medicine, such as cardiology, radiology, urology, interventional pain management, and internal medicine. The use of needles and needle systems in invasive procedures in various medical fields has become routine due, in part, to the ability of needles to pass through most tissues without causing significant destruction to the tissues.

Conventional needles have an orifice or a port at the distal tip of the needle. Distal tips of needles are more liable to clog as the tip of the needle is used for penetration of tissue to access the site of treatment. Additionally, there exists a greater chance of leakage of the agent being delivered, using conventional needles.

Another widely-used type of needle system includes a system that employs a catheter and guide member. Such needle systems generally include a small guide member (e.g., guide wire) which is used to guide a larger hollow catheter to a target area (e.g., a vessel, body cavity, tissue, or organ) within a human or animal body. Such needle systems are efficient for both therapeutic and diagnostic purposes.

It is also desirable to reduce a risk of internal injury from a sharp blade or edge of a traditional injection system or by providing a leading edge that is at least not as sharp, if not smooth, rounded, or flat. The leading edge may also be made of a material that is more yielding if a dense surface is encountered.

### SUMMARY

Various embodiments comprise a safety neural injection system comprising an at least partially hollow cannula being defined by a first inside diameter, a first outside diameter, a first length, and an open distal end in fluid communication with the inside and outside of the hollow cannula; a side port located coaxially along the hollow cannula in fluid communication between the inside and the outside of the hollow cannula; and a flexible stylet with a shaped tip, wherein the flexible stylet is capable of being releasably locked in a first position within the hollow cannula and extends at least the first length of the hollow cannula; wherein the hollow cannula proximate to the distal end includes a rigid bend so as to facilitate placement of the distal end at a target site. Further embodiments are characterized by the first position of the shaped tip of the flexible stylet being in a flush position relative to the open distal end of the hollow cannula. Further embodiments comprise a side port. In various further embodiments, the stylet is retractable. Further embodiments comprise a kit comprising the safety neural injection system of as herein described and an agent to be injected.

Further embodiments comprise a method of treatment for an individual in need thereof comprising locating a target site of treatment in the individual; adjusting the safety neural injection system of as described above; such that said system is positioned relative to said individual at said site and orientation; inserting at the target site at least a portion of said safety neural injection system; maneuvering the safety neural injection system inside said individual using the rigid bend proximate to the distal end to facilitate placement of said safety neural injection system at said target site; and treating said individual.

Various further embodiments comprise an injection system comprising means for locating a target site for treatment in an individual in need thereof; means for adjusting the safety neural injection system as herein described; such that said system is positioned relative to said individual at said site and orientation; means for inserting into the individual at least a portion of the safety neural injection system as herein described, wherein the hollow cannula, of the safety neural injection system as herein described, proximate to the distal end includes a rigid bend so as to facilitate placement of the distal end at the target site; means for maneuvering the distal end of the safety neural injection system inside said individual proximate to the site; and means for treating the individual.

Certain embodiments of the invention concern a safety neural injection system comprising:an at least partially hollow cannula being defined by a first inside diameter, a first outside diameter, a first length, and an open distal end in fluid communication with the inside and outside of the hollow cannula; a side port located coaxially along the hollow cannula in fluid communication between the inside and the outside of the hollow cannula; and a flexible stylet with a shaped tip, wherein the flexible stylet is capable of being releasably locked in a first position within the hollow cannula and extends at least the first length of the hollow cannula; wherein the hollow cannula proximate to the distal end includes a rigid bend so as to facilitate placement of the distal end at a target site, wherein the stylet and the hollow cannula define at least one channel.

In further embodiments the safety neural injection system further comprises one or more of the following, a connector, a wire, insulation, or a probe.

In embodiments of the safety neural injection system comprising a wire, the wire may be adapted to be connected about the connector with an instrument. In embodiments of the safety neural injection system comprising a connector, the connector may be a plug and use connector.

In embodiments of the invention concerning the position of the flexible stylet, in specific embodiments the first position is characterized by the shaped tip of the flexible stylet being in a flush position relative to the open distal end of the hollow cannula. In other specific embodiments, the first position is characterized by the shaped tip of the flexible stylet protruding beyond the first length.

In still further embodiments of the invention concerning a stylet, the stylet may itself further comprise a side port. In additional embodiments of the invention the stylet is retractable.

Additional embodiments of the invention contemplate an agent delivered via the side port attached to the cannula. In such embodiments the agent may be any agent. In particular embodiments, the agent selected from the group consisting of a therapeutic agent, a diagnostic agent or a prophylactic agent. In specific embodiments wherein a therapeutic agent is contemplated the therapeutic agent is anesthesia.

In alternative embodiments of the invention, a sideport may not exist in the cannula and the interference fit of the stylet within the cannula is such that medicament is capable of flowing out of the distal end at the target site between the stylet and the cannula.

In still other alternative embodiments of the invention, there is no channel and the interference fit of the stylet within the cannula is such that medicament is capable of flowing out of the distal end at the target site between the stylet and the cannula.

Other embodiments of the invention concern a method of injecting a medicament at a neuronal tissue comprising the steps of: locating a target site for administration; adjusting the safety neural injection system; such that said system is positioned relative to said site; inserting at the target site at least a portion of said safety neural injection system; maneuvering the safety neural injection system using the rigid bend proximate to the distal end to facilitate placement of said safety neural injection system at said target site; and administering said agent about said target site.

In certain embodiments concerning the aforementioned method, the target is a site selected from a group consisting of the digestive system, the circulatory system, nervous system, muscular system, skeletal system, respiratory system, urinary system, reproductive system, excretory system, endocrine system, immune system of the human body.

In such other embodiments of the invention, the agent is may be selected from the group consisting of a therapeutic agent, a diagnostic agent or a prophylactic agent. In particular aspects of the invention, wherein the agent is a therapeutic agent, the agent may be anesthesia. In other embodiments of the invention the agent is a biopsy needle. In still other embodiments of the invention, the agent is used for stimulating a tissue about the site of the site. In such embodiments, stimulating the tissue may comprise ablating at least a portion of the tissue.

In further aspects of a method of injecting a medicament at a neuronal tissue the method may comprise maneuvering the distal end of the injection system by the use of a real-time imaging device. In such aspects, the implementation of a real-time imaging device may permit a user to determine the exact location of the distal end at the target site.

In further aspects of a method of injecting a medicament at a neuronal tissue, the target site is a site selected from a group consisting of the digestive system, the circulatory system, nervous system, muscular system, skeletal system, respiratory system, urinary system, reproductive system, excretory system, endocrine system, immune system of the human body. In such embodiments, administering an agent may be accomplished with a stylet extending through the cannula. In certain aspects, the stylet can function as a safety measure during insertion of the cannula into the subject. In certain further aspects, the fit between the stylet and the cannula is such that a medicament is capable of passing between the cannula and the stylet.

Other embodiments of the invention comprise a kit of the aforementioned safety neural injection system.

Although the present invention is described with several embodiments, various changes and modifications may be suggested to one skilled in the art. In particular, the present invention is described with reference to certain polymers and materials and methods of processing those materials, but may apply to other types of processing or materials with little alteration and similar results. Furthermore, the present invention contemplates several process steps that may be performed in the sequence described or in an alternative sequence.

### BRIEF DESCRIPTION OF THE DRAWINGS

Figure 1 is an illustration of an embodiment of a safety neural injection system in a disassociated perspective;

Figure 2 is an illustration of an embodiment of a safety neural injection system in an associated perspective;

Figures 3A-3E are illustrations of embodiments of distal tips of hollow cannulas associated with shaped tips of flexible stylets; and,

Figure 4 is an illustration of an alternate embodiment of a safety neural injection system in a disassociated perspective.

Figure 5 is an illustration of an embodiment of a neural injection system with a cannula.

Figure 6 is an illustration of an alternate embodiment of a neural injection system with an alternate cannula and an alternate mating stylet in an associated manner.

### List of Reference Numerals

cannula 10
flexible stylet 20
cannula shaft 34
insulation 36
a rigid bent portion 38
distal tip 40
hub 42
index mark 43
stylet mating hub 44
distal opening 46
side port 48
index notch 50
stylet hub 52
shaped tip 56
stylet receiving notch 60
cannula 110
flexible stylet 120
hollow cannula shaft 134
rigid bent portion 138
distal tip 140
hub 142
index mark 143
stylet mating hub 144
distal opening 146
stylet hub 152
stylet receiving notch 160
cannula 170
reservoir 172
cannula shaft 174
stylet mating hub 176
stylet hub 178
second stylet hub 182
mating stylet 184
distal opening 186
distal tip 188
spray marks 190
cannula 200
hollow cannula shaft 204
stylet mating hub 206
second stylet hub 212
mating stylet 214
distal opening 216
distal tip 218

### DETAILED DESCRIPTION

### A. Introduction

The present invention relates generally to an injection system comprising a hollow cannula with an interior bore and with a proximal and distal end, the distal end being inserted into a subject and a flexible stylet with a shaped tip capable of being inserted into the interior bore of the hollow cannula. The invention further comprises a gas or fluid reservoir in fluid communication with the hollow cannula.

In one embodiment of the invention, a cannula having a reservoir attached to the cannula hub is provided wherein a gas or a fluidic therapeutic agent would be stored and dispensed into an insertion path along the hollow bore of a cannula having a stylus inserted into the hollow bore via a mating hub. In a second embodiment of the invention, the reservoir is attached to hub of a cannula having a hollow bore and having a stylus inserted through the hollow bore of cannula, wherein the stylus is permanently affixed to the cannula hub. In either embodiment, the reservoir is preferably in fluid connection with the cannula hub. In either embodiment, as an alternative to a gas or fluidic therapeutic reservoir, a vacuum source may be contemplated to aid in aspiration of liquid or cellular matter from a subject having the distal end of the cannula inserted into a cavity of the subject.

In the various embodiments of the invention, the reservoir may be in fluid connection to the cannula hub such that a fluidic therapeutic such as a liquid medicament may enter the hollow cannula at the desired time, typically after insertion of the distal end of the cannula into a cavity of the subject. The movement may be controlled such as via a plunger of a syringe, manual pressure placed on the reservoir via the hands of a clinician, pressure placed on the reservoir via a hydraulic pumping mechanism or a piston type pumping mechanism. The movement may also be controlled by gravity such as in the case wherein the reservoir is an i.v. bag containing a medicament suspended above the subject.

Various embodiments comprise a safety injection and treatment systems and related methods with at least one benefit of enhanced injection characteristics, increased operational efficiency, reduced cost per unit, reduced incidence of injury through intraneural or intravascular injection, reduced incidence of injury through pricking or piercing, or the like.

Various embodiments comprise a safety injection system include an at least partially hollow cannula. The cannula is defined by a first inside diameter, a first outside diameter, a first length, a side port located coaxially along the cannula for fluid communication between the inside and the outside of the hollow cannula, and an open distal end. The system also includes a flexible stylet with a shaped tip, wherein the flexible stylet is capable of being releasably locked in a first position within the hollow cannula and extends at least the first length of the hollow cannula. The hollow cannula is proximate to the distal end includes a rigid bend so as to facilitate placement of the distal end near a target region during system administration.

Various embodiments also includes methods and devices that are designed for injection of minute amounts of a fluid therapeutic, diagnostic or a prophylactic agent into tissue or a body wall, for example, an interior body wall. Additionally, the target site is located at sites or organs located in the human digestive system, the circulatory system, nervous system, muscular system, skeletal system, respiratory system, urinary system, reproductive system, excretory system, endocrine system, immune system of the human body is envisioned using the present invention. The amount of the agent to be administered according to the invention method will vary depending upon the goal to be accomplished, the size and age of the subject, the pharmacokinetics of the injectate, and the like.

Various embodiments are designed for treatment of a target tissue(s) at a target site. In an embodiment, treatment of a tissue may be at least one of probing, ablation, stimulating, or the like. In general, treatments capable with various embodiments can be any treatment common in the art and should not be limited by the present disclosure.

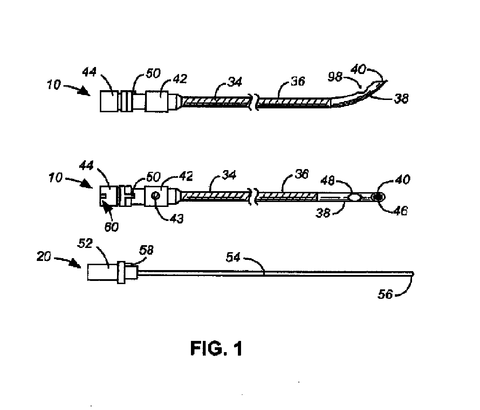

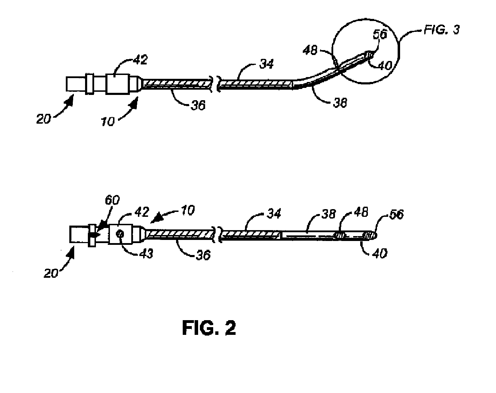

The principles of the present invention and their advantages are best understood by referring to Figures 1 and 2 of the drawings, like numerals being used for like and corresponding parts of the various drawings.

### B. Definitions

The following definitions and explanations are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3^{rd} Edition.

The term "attached," or any conjugation thereof describes and refers the at least partial connection of two items.

The term "medicament(s)" means and refers to all types of fluidic substances that have a beneficial, desired or therapeutic effect. Non-limiting examples of medicaments suitable for use in the invention methods include anesthesia, biologically active agents, such as small molecule drugs, proteinaceous substances, polynucleotides or nucleic acids (e.g., heterologous DNA, or RNA) and vectors, liposomes, and the like, containing such nucleic acids or polynucleotides, as well as liquid preparations or formulations thereof.

The term "medical instrument" means and refers to any item, instrument or structure capable of connecting to a catheter, such as, but not limited to a stimulation device, tubing, piping, a medicament delivery system, a meter, a liquid repository (e.g., an I.V. bag), a syringe, or the like.

The term "normal insertion procedure" means and refers to a typical surgical or insertion procedure as disclosed in Heavner et al., "Sharp Versus Blunt Needle: A Comparative Study of Penetration of Internal Structures and Bleeding in Dogs", 2003, World Institute of Pain, Pain Practice, 3:3, 226-231.

The term "stylet" means and refers to a small poniard. Stylets of the preset invention are capable of being hollow, but such is not required.

Other than in the operating examples, or where otherwise indicated, all numbers expressing quantities of ingredients or reaction conditions used herein are to be understood as modified in all instances by the term "about".

### C. Therapeutic Uses

In certain embodiments of the invention it is desirable to deliver a therapeutic agent to a subject through the use of a hollow cannula. Any agent that can be injected through a needle can be delivered using the inventive method. Typical agents might include saline solution, drugs, small molecules, pharmaceutical agents, diagnostic agents, biological molecules, proteins, peptides, antibodies, polynucleotides, RNA, DNA, viruses, cells, and combinations thereof. Agents may range in size from small organic molecules to macromolecules such as DNA to intact cells. The agent to be delivered to the injection site may be therapeutic (e.g., chemotherapeutic drug, antibiotic), prophylactic (e.g., vaccine), or diagnostic (e.g., contrast agent for magnetic resonance imaging, labeled metabolite). Therapeutic prophalytic or diagnostic agents to be delivered may also include biological molecules such as proteins, peptides, polynucleotides, and oligonucleotides. Examples of proteins or peptides include insulin, cytokines, growth factors, erythropoeitin, antibodies, antibody fragments, etc. Polynucleotides may be delivered for gene therapy and anti-sense therapy. In addition to drugs, small molecules, and biological molecules, the invention may be used to deliver viruses and cells, for example viruses for gene therapy delivery and cells for stem cell therapy delivery. In general, the therapeutic agent to be delivered will be in a liquid or amorphous form such as an ointment or cream for delivery through a hollow cannula.

In certain other embodiments of the invention the hollow cannula and stylet may be used for removal of tissue or liquids from a subject, such as in the use of paracentesis, thoracentesis, imaging via a camera attached or a part of the distal end of the stylet, surgical techniques such as a laser or other cutting instrument attached or a part of the distal end of the stylet, an electrical stimulator at the distal end of the stylet, a dye delivery or radio-isotope delivery system for diagnostic purposes, light delivery from a light emitting mechanism at the distal end of the stylus to visualize tissue or cells and the like.

Various embodiments may include methods of use of a safety neural injection system. An embodiment of a method may include locating a site for treatment in a patient; inserting into a patient at least a portion of a safety neural injection system comprised of an at least partially hollow cannula being defined by a first inside diameter, a first outside diameter, a first length, a side port located coaxially along the hollow cannula for fluid communication between the inside and the outside of the hollow cannula, and an open distal end; a flexible stylet with a shaped tip, wherein the flexible stylet is capable of being releasably locked in a first position within the hollow cannula and extends at least the first length of the hollow cannula; wherein the hollow cannula proximate to the distal end includes a rigid bend so as to facilitate placement of the distal end at the treatment site, maneuvering the distal end of the safety neural injection system inside a patient proximate to the site; and treating the patient.

Further embodiments of a method may include stimulating a tissue. Further embodiments of a method may include ablating at least a portion of the tissue about a site. Other embodiments comprise probing a tissue. And yet further embodiments of a method may include preparing a patient for administering a medicament.

In an embodiment of an administration of a block, such as a nerve block, the method may comprise preparing the patient. In an embodiment, preparing the patient may comprise placing a patient in a supine position or extended position, without a pillow, with the patient's head in a neutral position.

In an embodiment of administration of a medicament, while standing on side of the body that is to be blocked, the physician tactilely locates the cricoid cartilage. In such an embodiment, the neural injection system may be inserted in a position approximately one finger breadth below the cricoid cartilage, between the carotid sheath and the trachea on the side to be blocked, while aiming slightly medially until bony contact is made with the ventral lateral side of the body of the seventh cervical vertebra. When the neural injection system is in said position, the anesthesia may be injected.

In another embodiment, there may be methods for injecting a medicament into tissue of a subject. The method may include inserting the distal portion of an embodiment into the tissue of the subject and causing a therapeutic amount of medicament to enter multidirectionally from a distal end into the tissue. In various embodiments, a stylet is inserted through the cannula to act as a buffer to protect tissue of the patient. In various embodiments, the tissue is neuronal tissue. However, various embodiments of the present invention can be used with any tissue.

In various embodiments, the inserted stylet is left in the cannula and the medicament is injected to the target tissue about or around the inserted stylet. In such embodiments, a channel can be formed in the cannula to allow passage of the medicament. In various other embodiments, an interference fit of the stylet and the cannula is such that the medicament passes about the stylet through the distal opening of the cannula.

### D. Medical Instruments

Exemplary, non-limiting embodiments of medical instrument, injection systems, and the like that can be modified according to various teachings include, but are not limited to, U.S. Pat. No. 6,949,087; U.S. Pat. No. 6,855,132; U.S. Pat. No. 6,558,353; U.S. Pat. No. 6,547,769; U.S. Pat. No. 6,387,163; U.S. Pat. No. 6,245,044; U.S. Pat. No. 5,871,470; U.S. Pat. No. 5,865,806; U.S. Pat. No. 5,836,914; U.S. Pat. No. 5,817,074; U.S. Pat. No. 5,800,445; U.S. Pat. No. 5,730,749; U.S. Pat. No. 5,669,882; U.S. Pat. No. 5,628,734; U.S. Pat. No. 5,573,519; U.S. Pat. No. 5,571,091; U.S. Pat. No. 5,480,389; U.S. Pat. No. 5,466,225; U.S. Pat. No. 5,336,191; U.S. Pat. No. 5,312,360; U.S. Pat. No. 5,304,141; U.S. Pat. No. 5,250,035; U.S. Pat. No. 5,242,410; U.S. Pat. No. 5,106,376; U.S. Pat. No. 4,994,034; U.S. Pat. No. 4,973,313; U.S. Pat. No. 4,629,450; U.S. Pat. No. 4,317,445; U.S. Pat. No. 4,308,875; U.S. Pat. No. 4,230,123; U.S. Pat. No. 3,856,009; U.S. Pat. No. 3,565,074; and, U.S. Pat. No. 2,922,420.

A cannula associated with various embodiments may be a cylindrical structure extending from a proximal end to a distal end. The length from the proximate end to the distal end, traveling along the length of the cannula is known as the first length. In various embodiments where a bend in the cannula exists, the first length may be longer than the linear distance from the proximate end to the distal end. In an embodiment, the cannula is of a generally constant circumference. The cannula is capable of being differentiated by an inside diameter and an outside diameter. In an embodiment, an outside diameter is between about 0.0355 to about 0.03600 mm and an inside diameter is between about 0.0230 to about 0.0245 mm. In an alternate embodiment, an outside diameter between about 0.0205 to 0.280 mm and an inside diameter between about 0.0155 to 0.0170 mm. In various embodiments, an inside diameter and an outside diameter are capable of being any desired length and any particular length should not be construed as a limitation on the scope of the appended claims.

In various embodiments, a shaped tip end or distal end of the at least partially hollow cannula may take various shapes. In an embodiment, the distal tip may be the traditional beveled angular plane shape. In another embodiment, the distal tip may be squared with the perpendicular of the lengthwise plane of the cannula. In another embodiment, the distal tip may be a partial bevel, wherein the leading portion of the shaped tip is of a traditional bevel form and the remainder of the tip is formed in a non-beveled shape, such as a stair step. A variety of shapes may be considered for use to achieve the desired results while still possessing an open end.

In various embodiments, a connector may be about the proximal end of the cannula. A connector may be used as an attachment means for attaching the cannula and an optional further medical instrument. The connection or connections at the connector end may be any type of connection common in the art, for example, and not by way of limitation, a luer lock connector, a threaded attachment, an interference fit attachment, a clamp, a system utilizing a dowel, two or more of the aforesaid in combination, or the like.

A flexible stylet of various embodiments may extend through at least a portion of the hollow portion of the cannula. In some embodiments, a stylet may be characterized by an outside diameter and a length extending at least from a proximal end to the distal end, representing the first length. The outside diameter, in various embodiments, is smaller than the first inside diameter of the cannula. In various embodiments, the stylet may be of a length that is longer than the first length. In some embodiments, the stylet comprises a side port an is at least partially hollow. In an embodiment, the stylet and the cannula define a passageway for passage of at least one medicament.

In various embodiments, the material of construction may permit the flexible stylet so as to bend within the hollow cannula to conform generally to its internal shape. In some embodiments, the material of construction of the flexible stylet may be of a polymer material. In some embodiments, the material will be made of a biocompatible material. Examples of such embodiments include materials such as polyethylene, polypropylene, and polyfluorocarbons. In some alternative embodiments, the material of construction may be of a metallic material. Examples of embodiments include steel alloys, titanium alloys, and aluminum. In various embodiments, the stylet exhibits elastic deformation in regards to insertion and removal from the cannula.

In various embodiments, the flexible stylet may be capable of being inserted into the cannula so that the distal tip of the stylet may be position within the cannula at any point. In an embodiment, the distal end of the stylet may be positioned so that the distal end of the stylet is equivalent with the distal end of the cannula in relation to the proximate end of the cannula. In an alternate embodiment, the distal end of the stylet may extend past the distal end of the cannula. In an alternate embodiment, the distal end of the cannula may extend past the distal end of the stylet.

In various embodiments, the flexible stylet may possess a shaped distal tip to support not only the "safety" function of providing a smooth, flat, or leading surface versus the potentially sharp or rigid edge of the distal tip of the hollow cannula but also provide the means for the leading tip of the system to be later retracted when the flexible stylet is partially or completely decoupled from the hollow cannula so as to retract the shaped tip of the flexible stylet from its leading position. In some embodiments, the shaped tip may be rounded, similar to a half-dome or a bullet. In some embodiments, the shaped tip may be flat in a manner that is squared with the length of the stylet. In some embodiments, the shaped tip may be a traditional bevel angle. In some embodiments, the shape of the tip will conform approximately to the shape of the distal end of the cannula. In some embodiments, the shaped end of the flexible stylet may be formed in a manner so as to form a flush and flat surface with the distal end of the cannula. In some embodiments, the shaped tip may be edged or pointed such that at least a portion of the distal edge of the stylet is capable of penetrating a tissue that a normal insertion procedure could not penetrate. In some embodiments, the shaped tip of the stylet is a point.

Various embodiments may fixedly connect, releasably connect, or leave unconnected the flexible stylet and the cannula. In another embodiment, the stylet is capable of sliding within the cannula. In another embodiment, the stylet is releasably secured within the cannula by a locking mechanism, such as, but not limited to a luer lock, an interference fit, a snap, screw threads, or the like. In an embodiment of a luer lock system, internal male luer threads are located in or about the stylet adjacent to receive and engage a cannula having female luer threads thereon. In other embodiments, the luer lock is reversed. In another embodiment, the cannula is welded to or otherwise fixedly connected to the cannula.

Further embodiments may comprise a cannula or stylet with multiple ports arranged in any orientation about the shaft. In an embodiment, a stylet may comprise, in application, a side port across a cannula and a side port across the stylet. In various embodiments, the side ports may be positioned such that reasonable alignment of the side ports occurs at a desired position of the stylet within the cannula such that a medicament may pass from across the reasonably aligned side ports.

Various embodiments may include a wire or other means of conveying stimulation to a target tissue. In an embodiment, the wire may extends along the cannula from about the proximal end to about the distal end of the distal tip. In another embodiment, the wire may be integral (attached to) with the cannula. In another embodiment, the wire may extend along the outside of the cannula. In another embodiment, a wire may extend along, through, or is integral with the stylet.

Design consideration that may be implemented with various embodiments include, but are not limited, to designing the wire and connector such that they may be utilized as a "plug and use" type of arrangement. A plug and use arrangement is beneficial because it reduces the complexity of the device and reduces loose wires. In an embodiment, the wire may be formed into the connector such that when the connector is connected to another medical instrument, the wire is able to communicate with the instrument. However, any connection common in the art that would allow the wire to communicate with a medical instrument may be contemplated within various embodiments.

Various embodiments may include insulation or at least one form insulation about the cannula, stylet, or wire. As may be appreciated by one of ordinary skill in the art, any material of construction that provides electrical or thermal insulation could be used for example, but not limited to, a plastic, a rubber, a metal, a non-metal, or the like. In some embodiments, the insulation covers the exterior of the hollow cannula along the entire first length. In some embodiments, the insulation covers the exterior of the hollow cannula until it reaches the rigid bend portion of the hollow cannula. In some embodiments, the insulation covers an exterior portion of the hollow cannula in between the hub and the rigid bend portion.

Various embodiments may include insulation around the hollow cannula, flexible stylet, or wire that is constructed of a material that permits differentiation between the insulation and the hollow cannula or flexible stylet material during real-time procedural use. Numerous procedures, for example, but not limited to, fluoroscopic guidance procedures, NMR procedures, X-ray procedures, direct viewing procedures, or the like, may be used during a medical procedure to determine the position of a safety neural injuection system and the target location. In such embodiments, a practitioner may choose an embodiment with an insulation coating wherein the absorptive or reflective difference between the insulation coating and the uninsulated portion of the safety neural injection system can be differentiated in real-time using the selected real-time viewing system. For example, a particular insulation may absorb the energy from a real-time viewing system and show up as a dark segment whereas the uninsulated portion may reflect the energy and appear to be a bright segment. In such embodiments, the differentiation in reflectivity and absorption may provide a method to determine the exact position of the shaped tip, the distal tip, or the side portal of the safety neural needle in relation to the treatment site, given that the relative distance from the insulation/non-insulation border.

### E. Specific Embodiments

Figure 1 illustrates an embodiment of a safety neural injection system 1 with a cannula 10 (viewed in both top and side perspectives) and a mating flexible stylet 20 in a disassociated manner. The cannula 10 is capable of being characterized as having a first length measured from the proximate end of the stylet mating hub 44 to the distal opening 46 of the distal tip 40. In this embodiment, the cannula 10 has a hollow cannula shaft 34 with a rigid bent portion 38 near the distal end and possesses insulation 36 lining part of the cannula shaft 34. Insulation 36 is capable of use to isolate a radio frequency (RF) portion of the cannula, to isolate an RF wire, and/or the like. However, various embodiments comprise a cannula without insulation.

In an embodiment, a side port 48 is located in the rigid bent portion 38 of the hollow cannula shaft 34 proximate to the distal tip 40 and distal opening 46. However, a side port is not present in various other embodiments. At the proximate end of the cannula 10, a hub 42 is endowed with an index mark 43 to visually inform the user of the direction of bend the rigid bent portion 38 is directed toward. Additionally, an index notch 50, located on the stylet mating hub 44, performs a similar function tactilely so as to inform the user of the directional perspective of the rigid bent portion 38. The stylet mating hub 44 also possesses a stylet receiving notch 60 so as to fixedly engage a flexible stylet 20 and directionally fix its position.

In this embodiment, the flexible stylet 20 is comprised of a stylet hub 52 with a stylet lock notch 58 shaped to mate with the stylet receiving notch 60 in a frictional manner, a stylet shaft 54, and a shaped stylet tip 56. The stylet shaft 54 is characterized by having a length of at least the first length associated with the first length of the cannula 10. The stylet shaft is also characterized by a material of construction that permits elastic return even after prolonged engagement within a hollow cannula 10 with a rigid bent portion 38.

Figure 2 illustrates another embodiment of a safety needle injection system wherein the flexible stylet 20 that extends beyond the first length of the cannula 10 possessing a shaped tip 56 is mated and engaged with a cannula 10 with a side port 48, a distal tip 40, a rigid bent portion 38, and insulation 36.

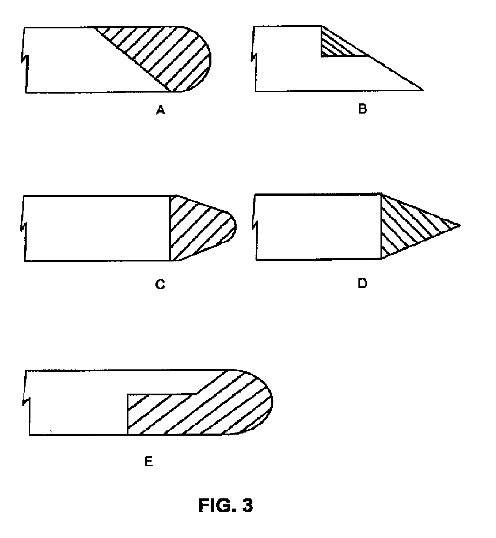

Figures 3A -3E illustrates more closely several embodiments of the distal tip 40 of the cannula 10 with the shaped tip 56 of the flexible stylet 20. Figure 3A shows an example of a shaped tip 56 as a rounded end extending beyond the end of the traditional bevel shape of the distal tip 40. Figure 3A also inherently demonstrates a flexible stylet 20 that has a stylet shaft 54 that is longer than the first length of the associated cannula 10 because the end of the flexible stylet 20 extends beyond the furthest distal point of the distal tip 40. Figure 3B shows an example of a partial bevel distal tip 56 at the distal end of a cannula 10 matched with tip of a flexible stylet 20, wherein the shape of the shaped tip 56 corresponds with the angle of the bevel portion of the distal tip 56 and forms a partially flush and flat surface at the open distal end 40. Such a combination of shapes that form a flush, beveled surface might be intended for use to prevent tearing and "grabbing" of the distal tip on parts of the body by the more squared portion of the distal tip 40. Figure 3C and 3D show a distal tip 40 in a squared form with flexible stylet shaped tips 56 extending beyond the first length. Figure 3C gives an example of a rounded point or "pen point" form for shaped tip 56. Figure 3D gives an example of a sharp leading point for shaped tip 56. Figure 3E is an illustration of the needle tip disclosed in US 5,810,788.

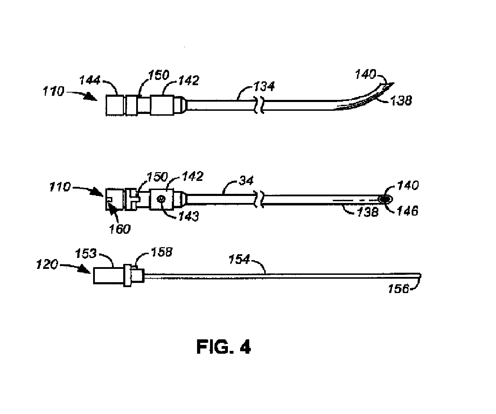

Figure 4 illustrates an alternate embodiment of a safety neural injection system with a cannula 110 (viewed in both top and side perspectives) and a mating flexible stylet 120 in a disassociated manner. The cannula 110 is capable of being characterized as having a first length measured from the proximate end of the stylet mating hub 144 to the distal opening 146 of the distal tip 140. In this embodiment, the cannula 110 has a hollow cannula shaft 134 with a rigid bent portion 138 near the distal end.

The proximate end of the cannula 110, a hub 142 is endowed with an index mark 143 to visually inform the user of the direction of bend the rigid bent portion 138 is directed toward. Additionally, an index notch 150, located on the stylet mating hub 144, performs a similar function tactilely so as to inform the user of the directional perspective of the rigid bent portion 138. The stylet mating hub 144 also possesses a stylet receiving notch 160 so as to fixedly engage a flexible stylet 120 and directionally fix its position.

In this embodiment, the flexible stylet 120 is comprised of a stylet hub 152 with a stylet lock notch 58 shaped to mate with the stylet receiving notch 160 in a frictional manner, a stylet shaft 154, and a shaped stylet tip 156. The stylet shaft 154 is characterized by having a length of at least the first length associated with the first length of the cannula 110. The stylet shaft is also characterized by a material of construction that permits elastic return even after prolonged engagement within a hollow cannula 110 with a rigid bent portion 138.

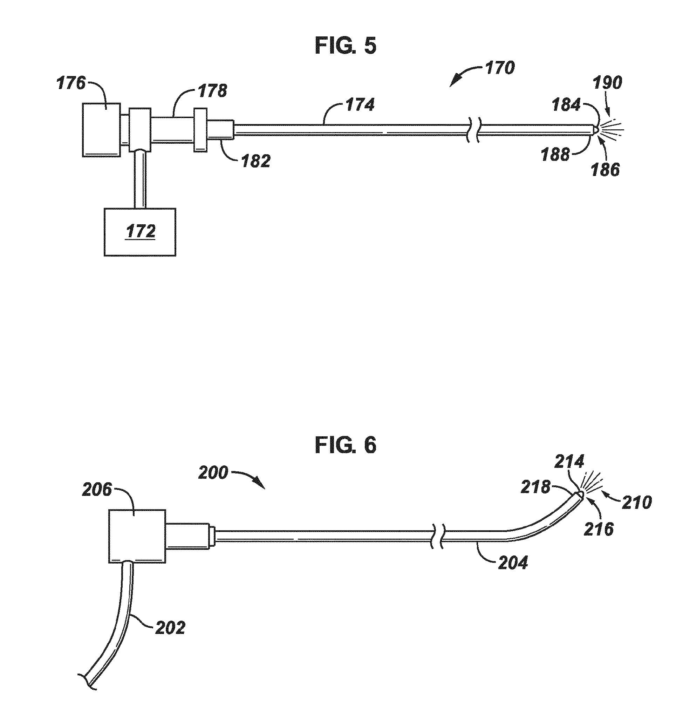

Figure 5 illustrates an alternate embodiment of a neural injection system with a cannula 170 and a mating stylet 184 in an associated manner. The cannula 170 is capable of being characterized as having a first length measured from the proximate end of the stylet mating hub 176 to the distal opening 186 of the distal tip 188. In this embodiment, the cannula 170 has a hollow cannula shaft 174 that is straight and at least semi rigid.

The proximate end of the cannula 170, a hub 178 is connected to a reservoir 172. Additionally, indexing on stylet hub 178 and second stylet hub 182 can be used to tactilely and/or visually inform the user of the location of the stylet within cannula 170.

In this embodiment, stylet 184 and cannula 170 define a fluid passageway wherein a user can inject a medicament as is shown by spray marks 190 out of distal opening 186 without removing stylet 184. Such an embodiment is capable of use when injecting a medicament around neuronal tissue that may be damaged by a cannula without the protection of the stylet. In this embodiment, stylet 184 extends beyond cannula shaft 174 and is capable of providing a buffer for neuronal tissue. In various other embodiments, the stylet extends through the cannula shaft.

Figure 6 illustrates an alternate embodiment of a neural injection system with a cannula 200 and a mating stylet 214 in an associated manner. The cannula 200 is capable of being characterized as having a first length measured from the proximate end of the stylet mating hub 206 to the distal opening 216 of the distal tip 218. In this embodiment, the cannula 200 has a hollow cannula shaft 204 that is bent.

The proximate end of the cannula 200, hub 206 is connected to a reservoir line 202 that is capable of supplying a medicament or other fluid, such as a liquid or a gas. Additionally, indexing on stylet hub 206 and second stylet hub 212 can be used to tactilely and/or visually inform the user of the location of the stylet within cannula 200.

In this embodiment, stylet 214 and cannula 200 define a fluid passageway wherein a user can inject a medicament as is shown by spray marks 210 out of distal opening 216 without removing stylet 214. Such an embodiment is capable of use when injecting a medicament around neuronal tissue that may be damaged by a cannula without the protection of the stylet. In this embodiment, stylet 214 extends beyond cannula shaft 204 and is capable of providing a buffer for neuronal tissue.

### F. Further Embodiments

The disclosure also includes the subject matter of the following numbered clauses:
1. A safety neural injection system comprising:
   an at least partially hollow cannula being defined by a first inside diameter, a first outside diameter, a first length, and an open distal end in fluid communication with the inside and outside of the hollow cannula;
   a side port located coaxially along the hollow cannula in fluid communication between the inside and the outside of the hollow cannula; and
   a flexible stylet with a shaped tip, wherein the flexible stylet is capable of being releasably locked in a first position within the hollow cannula and extends at least the first length of the hollow cannula; wherein the hollow cannula proximate to the distal end includes a rigid bend so as to facilitate placement of the distal end at a target site, wherein the stylet and the hollow cannula define at least one channel.
2. The system of clause 1, wherein the first position is characterized by the shaped tip of the flexible stylet being in a flush position relative to the open distal end of the hollow cannula.
3. The system of clause 1, wherein the shaped tip is configured so as to form a flat plane across the open distal end.
4. The system of clause 1, wherein the first position is characterized by the shaped tip of the flexible stylet protruding beyond the first length.
5. The system of clause 1, further comprising an agent selected from the group consisting of a therapeutic agent, a diagnostic agent or a prophylactic agent.
6. The system of clause 5, wherein said therapeutic agent is anesthesia.
7. The system of clause 1, further comprising a connector.
8. The system of clause 1, further comprising a probe.
9. The system of clause 5, further comprising a wire, wherein the wire is adapted to be connected about the connector with an instrument.
10. The system of clause 1, wherein the stylet further comprises a side port.
11. The system of clause 1, wherein there is no sideport in the cannula and the interference fit of the stylet within the cannula is such that medicament is capable of flowing out of the distal end at the target site between the stylet and the cannula.
12. The system of clause 1, wherein there is no channel and the interference fit of the stylet within the cannula is such that medicament is capable of flowing out of the distal end at the target site between the stylet and the cannula.
13. A method of injecting a medicament at a neuronal tissue comprising the steps of:
   locating a target site for administration;
   adjusting the safety neural injection system of any of clauses 1 to 12 (for example clause 1) such that said system is positioned relative to said site;
   inserting at the target site at least a portion of said safety neural injection system;
   maneuvering the safety neural injection system using the rigid bend proximate to the distal end to facilitate placement of said safety neural injection system at said target site; and
   administering said agent about said target site.
14. The method of clause 13, wherein the agent is selected from the group consisting of a therapeutic agent, a diagnostic agent or a prophylactic agent.
15. The method of clause 13, wherein the therapeutic agent is anesthesia.
16. The method of clause 13, wherein said agent is a biopsy needle.
17. The method of clause 13, wherein the step of stimulating the tissue comprises ablating at least a portion of the tissue.
18. The method of clause 13, wherein said target site is a site selected from a group consisting of the digestive system, the circulatory system, nervous system, muscular system, skeletal system, respiratory system, urinary system, reproductive system, excretory system, endocrine system, immune system of the human body.
19. A kit comprising the safety neural injection system of clause 1 and an agent to be administered.
20. The kit of clause 29, wherein said agent is selected from a group consisting of a therapeutic agent, a diagnostic agent or a prophylactic agent.

## Claims

1. A safety neural injection system comprising:
an at least partially hollow cannula being defined by a first inside diameter, a first outside diameter, a first length, and an open distal end in fluid communication with the inside and outside of the hollow cannula;
a side port located coaxially along the hollow cannula in fluid communication between the inside and the outside of the hollow cannula; and
a flexible stylet with a shaped tip, wherein the flexible stylet is capable of being releasably locked in a first position within the hollow cannula and extends at least the first length of the hollow cannula; wherein the hollow cannula proximate to the distal end includes a rigid bend so as to facilitate placement of the distal end at a target site, wherein the stylet and the hollow cannula define at least one channel.

2. The system of claim 1, wherein the first position is **characterized by** the shaped tip of the flexible stylet being in a flush position relative to the open distal end of the hollow cannula.

3. The system of claim 1 or claim 2, wherein the shaped tip is configured so as to form a flat plane across the open distal end.

4. The system of claim 1, wherein the first position is **characterized by** the shaped tip of the flexible stylet protruding beyond the first length.

5. The system of any preceding claim, further comprising an agent selected from the group consisting of a therapeutic agent, a diagnostic agent or a prophylactic agent.

6. The system of claim 5, wherein the agent is a therapeutic agent.

7. The system of claim 5 or claim 6, wherein said therapeutic agent is anesthesia.

8. The system of any preceding claim, further comprising a connector.

9. The system of any preceding claim, further comprising a probe.

10. The system of any preceding claim, further comprising a wire, wherein the wire is adapted to be connected about the connector with an instrument.

11. The system of any preceding claim, wherein the stylet further comprises a side port.

12. The system of any preceding claim, wherein there is no sideport in the cannula and the interference fit of the stylet within the cannula is such that medicament is capable of flowing out of the distal end at the target site between the stylet and the cannula.

13. The system of any preceding claim, wherein there is no channel and the interference fit of the stylet within the cannula is such that medicament is capable of flowing out of the distal end at the target site between the stylet and the cannula.

14. A kit comprising the safety neural injection system of any of claims 1 to 13 and an agent to be administered.

15. The kit of claim 14, wherein said agent is selected from a group consisting of a therapeutic agent, a diagnostic agent or a prophylactic agent.
